Europäisches Patentamt

European Patent Office

Office européen des brevets

(11) Veröffentlichungsnummer: **0 424 767 A2**

# EUROPÄISCHE PATENTANMELDUNG

(21) Anmeldenummer: 90119672.5

(22) Anmeldetag: **15.10.90**

(51) Int. Cl.5: **C07C 31/125**, C07C 69/80,
C07C 69/44, C07C 67/08,
C07C 29/16, C07C 1/24,
C07C 2/30, C07C 11/02,
C08K 5/11, C08K 5/12

(30) Priorität: **27.10.89 DE 3935796**

(43) Veröffentlichungstag der Anmeldung:
**02.05.91 Patentblatt 91/18**

(84) Benannte Vertragsstaaten:
**BE DE ES FR GB IT NL SE**

(71) Anmelder: **BASF Aktiengesellschaft**
**Carl-Bosch-Strasse 38**
**W-6700 Ludwigshafen(DE)**

(72) Erfinder: **Hoffmann, Herwig, Dr.**
**Knietschstrasse 21**
**W-6710 Frankenthal(DE)**
Erfinder: **Roeper, Michael, Dr.**
**Albert-Schweitzer-Strasse 10**
**W-6706 Wachenheim(DE)**
Erfinder: **Schulze, Joachim, Dr.**
**Pielachtalstrasse 26**
**W-6700 Ludwigshafen(DE)**
Erfinder: **Strohmeyer, Max, Dr.**
**Woogstrasse 41**
**W-6703 Limburgerhof(DE)**
Erfinder: **Ulrich, Bernhard, Dr.**
**Auf dem Heyer 28**
**W-6719 Bockenheim(DE)**

(54) **Decanolgemische und daraus erhältliche Di-decylester-Gemische und ihre Verwendung als Weichmacher.**

(57) Di-decylphthalat-Gemisch bestehend aus Di-i-decylphthalaten, erhalten durch die folgenden Verfahrensschritte
a) Dimerisierung von einem Butengemisch, aus dem Butadien und Isobuten abgetrennt sind, mit einem Katalysator aus Alkylaluminiumdihalogenid und einer Verbindung eines Metalls der Gruppe VIII zu einem octengemisch,
b) Hydroformylierung und Hydrierung des Octengemisches zu einem Nonanolgemisch,
c) Dehydratisierung des Nonanolgemischs unter Bildung eines Nonengemisches,
d) Hydroformylierung und Hydrierung des Nonengemisches unter Bildung eines Decanolgemisches und
e) Veresterung mit Phthalsäureanhydrid.
Die neuen Phthalate zeichnen sich durch vorteilhafte Weichmachereigenschaften aus.

EP 0 424 767 A2

## DECANOLGEMISCHE UND DARAUS ERHÄLTLICHE DI-DECYLESTER-GEMISCHE UND IHRE VERWENDUNG ALS WEICHMACHER

Die Erfindung betrifft neue Decanolgemische und daraus erhältliche Di-decylester-Gemische, die als Weichmacher Verwendung finden und die über eine 2-fache Hydroformylierung aus Octen, das seinerseits durch Butendimerisierung hergestellt ist, erhalten werden.

Aus DE-C2-28 55 421 ist bekannt, daß Di-nonylphthalate, die man durch Hydroformylierung von durch Dimerisierung von Buten hergestelltem Octen und Veresterung mit Phthalsäureanhydrid erhält, sehr gute Eigenschaften als PVC-Weichmacher aufweisen.

Es wurde nun überraschenderweise gefunden, daß diese Eigenschaften insbesondere die Viskosität und Fließfähigkeit, sich noch weiter verbessern lassen mit Estern aus einem Decanolgemisch und Phthalsäureanhydrid oder Adipinsäure, insbesondere einem Di-decylphthalat-Gemisch, bestehend aus Di-i-decylphthalaten bzw. Di-i-decyladipaten, erhalten durch die folgenden Verfahrensschritte

a) Dimerisierung von einem Butengemisch, aus dem Butadien und Isobuten abgetrennt sind, mit einem Katalysator aus Alkylaluminiumdihalogenid und einer Verbindung eines Metalls der Gruppe VIII oder mit einem Trägerkatalysator, der Nickel und Molybdän auf Magnesiumsilikat enthält, zu einem Octengemisch,

b) Hydroformylierung und Hydrierung des Octengemisches zu einem Nonanolgemisch,

c) Dehydratisierung des Nonanolgemischs unter Bildung eines Nonengemisches,

d) Hydroformylierung und Hydrierung des Nonengemisches unter Bildung eines Decanolgemisches und

e) Veresterung mit Phthalsäureanhydrid bzw. Adipinsäure.

Dabei ist die homogene Katalyse bei der Stufe a) mit einem Katalysator aus Alkylaluminiumdihalogenid und einem Nickelsalz einer Fettsäure bevorzugt.

Ein Nickel und Molybdän auf Magnesiumsilikat enthaltender Katalysator enthält z.B. ca. 55 % NiO, ca. 5 % $MoO_3$, ca. 15 % MgO und ca. 25 % $SiO_2$.

Octen erhalten durch Dimerisierung von Butenen mit einem Katalysator aus Alkylaluminiumdihalogenid und einer Verbindung eines Metalls der Gruppe VIII, z.B. dem Nickelsalz einer Carbonsäure wie es z.B. in EP-81-0024971 im einzelnen beschrieben ist, wird in an sich bekannter Weise bei 150 bis 190° C und 260 bis 280 bar mit einem Cobalt-Katalysator hydroformyliert.

Im einzelnen geht man dabei z.B. so vor, daß man das Olefin in einer zweistufigen Reaktorkaskade bei 185 bis 190° C und 260 bis 280 bar mittels eines Cobaltkatalysators (z.B. Cobaltacetat) bei einer Verweilzeit von ca. 180 min hydroformyliert. Nach Abtrennung des Katalysators durch oxidative Entcobaltung und Rückführung in die Synthese, wird das Hydroformylierungsprodukt der Hydrierung unterworfen.

Dazu wird das cobaltfreie Hydroformylierungsprodukt bei 150 bis 210° C und 200 bis 260 bar über einen Festbettkatalysator in Rieselfahrweise hydriert. Beispiele für Hydrierkatalysatoren sind Kontakte auf Basis von Kupfer, Kupferchromit, Nickel oder Cobalt auf einen Träger bzw. als Vollkontakt.

Der Hydrieraustrag wird in an sich bekannter Weise aufgearbeitet. In einer ersten Kolonne werden über Kopf Kohlenwasserstoffe und Wasser abgetrennt, am Kopf der zweiten Kolonne fällt der Reinalkohol an. Der Sumpfaustrag der Reinalkoholkolonne kann gegebenenfalls in einer Rückstandskolonne von Alkoholresten befreit werden. Als Produktströme erhält man demnach neben dem Reinalkohol (100 Teile) noch 5 bis 20 Teile Kohlenwasserstoffe und einen hochsiedenden Rückstand (5 bis 10 Teile).

Das so erhaltene Nonanol wird dann in an sich bekannter Weise zum Nonen bei Temperaturen von 300 bis 400° C über $Al_2O_3$ als Katalysator dehydratiiert, wie dies z.B. bei J. Boeseken und C.J.A. Hanegraaff, Rec.Trav. Chim. 61, 69 (1942) beschrieben ist.

Das erhaltene Nonen wird in der gleichen Weise und unter im wesentlichen gleichen Bedingungen wie für Octen beschrieben erneut hydroformyliert und hydriert.

Das schließlich erhaltene neue Decanolgemisch wird dann in ebenfalls an sich bekannter Weise mit Phthalsäureanhydrid bzw. Adipinsäure z.B. im Molverhältnis Decanol zu Phthalsäureanhydrid 2:1 verestert, wie dies z.B. in Ullmanns Encyclopädie der technischen Chemie (1962) 13. Band, S. 728 bis 731 beschrieben ist. Die Eigenschaften des bekannten Diisodecylphthalats sind im 18. Band S. 559 beschrieben.

Gegenüber dem bekannten Diisodecylphthalat, das aus hydroformyliertem Trimerpropylen erhalten wurde, zeichnet sich das erfindungsgemäße Diisodecylphthalat durch geringere Viskosität und geringere Flüchtigkeit aus.

Beispiele

Okten:

Aus 207,2 g Raffinat II, das 70 % Butene enthält, erhält man unter Rühren in Gegenwart von 0,0227 g Nickelethylhexanoat und 0,1238 g Ethylaluminiumdichlorid bei 40 bis 45°C und 6 bis 10 bar 207,5 g Rohgemisch. Nach der Zerstörung des Katalysators mit 0,0828 g $NH_3$ (100 %ig und 15 %ige Natronlauge) erfolgt Schichtentrennung und anschließend eine Destillation.

Bei der Destillation werden bei 40°C und 3 bar 89,8 g Kohlenwasserstoffe gewonnen und bei der anschließenden Reinfraktionierung 100 g Okten bei 86°C und 350 mbar abdestilliert. Das erhaltene Okten enthält ca. 5 % n-Okten, 58 % monoalkylverzweigte und 37 % dialkylverzweigte Oktene.

Nonanol

91 g Okten werden bei 280 bar und 185°C mit Oxogas (ca. 50 % CO und 50 % $H_2$) in Gegenwart von ca. 0,15 % Kobaltcarbonylwasserstoff oxidiert und nach der Entkobaltung, Entgasung und Schichtentrennung wird die org. Phase hydriert. Die Hydrierung erfolgt in Gegenwart von Wasserstoff bei 280 bar und 186°C an einen Nickel/Molybdän-Katalysator bei einer Belastung von etwa 0,14 l/l x h.

Der Hydrieraustrag (= 100 g) enthält etwa 0,6 % Olefin, 5,4 % Oktane, 87 % Nonanol und etwa 7 % Rückstand. Die CO-Zahl liegt im Austrag bei etwa 0,3.

Aus 130 g Hydrieraustrag werden bei einer Sumpftemperatur von etwa 152°C, einer Kopftemperatur von ca. 48°C und einem Kopfvakuum von 150 mbar 10 g Octan abdestilliert. Bei einer Sumpftemperatur von etwa 188°C, einer Kopftemperatur von 150°C und einem Kopfvakuum von ca. 150 mbar werden 100 g Nonanol abdestilliert. Das Rückflußverhältnis beträgt bei der KW-Abtrennung ca. 3 und beim Reinalkohol = 1, Rückstand = 20 g.

Das so erhaltene Nonanol enthält etwa 5 % n-Nonanol, 58 % monoalkylverzweigte und 37 % dialkylverzweigte Nonanole.

Nonen

Aus 120 g Nonanol wird bei einem Druck von 1,4 bar und einer Temperatur von 350°C an einem $Al_2O_3$ Katalysator Wasser abgespalten. Der Durchsatz beträgt 0,42 l/l x h. Nach Abtrennung der oberen Phase werden 100 g Rohnonen mit folgender Zusammensetzung erhalten:

94,2 % Nonen
4,4 % Rückstand
1,4 % gesättigte Kohlenwasserstoffe

Dekanol

85 g Nonen werden bei 183 bis 185°C und 280 bar mit Oxogas (ca. 50 % CO und 50 % $H_2$) in Gegenwart von ca. 0,15 % Kobaltcarbonylwasserstoff oxidiert und nach der Entkobaltung, Entgasung und Schichtentrennung wird die org. Phase hydriert. Die Hydrierung erfolgt in Gegenwart von Wasserstoff bei 280 bar und 186°C an einem Nickel-Molybdän-Katalysator bei einer Belastung von 0,14 l/l x h.

Der Hydrieraustrag (= 100 g) enthält etwa 0,6 % Olefin, 5,8 % Nonane, 84,6 % Dekanol und 9 % Rückstand. Die CO-Zahl liegt im Austrag bei etwa 0,6.

Aus 130 g Hydrieraustrag werden bei einer Sumpftemperatur von etwa 142°C, einer Kopftemperatur von etwa 84°C und einem Kopfvakuum von 54 mbar 8 g Nonan abdestilliert. Bei einer Sumpftemperatur von etwa 166°C, einer Kopftemperatur von etwa 105°C und einem Kopfvakuum von 10 mbar werden 100 g Dekanol abdestilliert. Das Rückflußverhältnis beträgt bei der KW-Abtrennung etwa 3 und beim Reinalkohol 1; Rückstand = 22 g.

Das Isodekanol besteht aus etwa 50 Isomeren mit 4 Hauptkomponenten zwischen 8 und 18 %. Der Verzweigungsgrad beträgt nur 1,64, während Isodekanol, hergestellt aus Trimerpropen, einen Verzweigungsgrad von 2,09 aufweist (bestimmt mit $^1$H-NMR-Analytik auf einem hochauflösenden 400 MHz-Gerät).

Diisodecylphthalat

37,3 g Phthalsäureanhydrid werden mit 95,7 g Isodecanol und 0,05 g Tetrabutyltitanat als Katalysator im Rührkolben bei 230°C verestert. Der Katalysator wird mit 0,5 %iger Sodalösung hydrolysiert, das entstandene Titandioxidhydrat mit der wäßrigen Phase abgetrennt und nochmals mit Wasser gewaschen. Der überschüssige Alkohol wird abdestilliert (17 g Rückalkohol) und der Rohester zur Entfernung der letzten Alkoholreste einer Wasserdampfdestillation unterzogen. Der Rohester wird bei 90°C und 70 mbar im Stickstoffstrom mit Aktivkohle behandelt und filtriert. Es entstehen 100 g Diisodecylphthalate.

|  | Diisodecylphthalat | |
|---|---|---|
|  | aus Decanol, erhalten durch Oxierung von Trimerpropylen | aus erfindungsgemäß erhaltenem Decanol |
| Viskosität<br>Flüchtigkeit (Langzeittest 7 Tage bei 100° C Weich PVC 60 Teile S-PVC K-Wert 70 40 Teile Weichmacher 1,2 Teile Ba-Zn-Stabilisator)<br>Fogging Test (3 h bei 100° C Reflexionswert) | 120 bis 130 mPa•s<br>ca. 0,54 %<br><br><br>86 % | 101,3 mPa•s<br>0,45 %<br><br><br>99 % |

**Ansprüche**

1. Decanolgemische, erhalten durch die folgenden Verfahrensschritte
   a) Dimerisierung von einem Butengemisch, aus dem Butadien und Isobuten abgetrennt sind, mit einem Katalysator aus Alkylaluminiumdihalogenid und einer Verbindung eines Metalls der Gruppe VIII oder mit einem Trägerkatalysator, der Nickel und Molybdän auf Magnesiumsilikat enthält, zu einem Octengemisch,
   b) Hydroformylierung und Hydrierung des Octengemisches zu einem Nonanolgemisch,
   c) Dehydratisierung des Nonanolgemischs unter Bildung eines Nonengemisches
   und
   d) Hydroformylierung und Hydrierung des Nonengemisches unter Bildung eines Decanolgemisches.

2. Di-decylester-Gemisch bestehend aus Di-i-decylphthalaten oder Di-i-decyladipaten, erhalten durch die folgenden Verfahrensschritte
   a) Dimerisierung von einem Butengemisch, aus dem Butadien und Isobuten abgetrennt sind, mit einem Katalysator aus Alkylaluminiumdihalogenid und einer Verbindung eines Metalls der Gruppe VIII oder mit einem Trägerkatalysator, der Nickel und Molybdän auf Magnesiumsilikat enthält, zu einem Octengemisch,
   b) Hydroformylierung und Hydrierung des Octengemisches zu einem Nonanolgemisch,
   c) Dehydratisierung des Nonanolgemischs unter Bildung eines Nonengemisches,
   d) Hydroformylierung und Hydrierung des Nonengemisches unter Bildung eines Decanolgemisches und
   e) Veresterung mit Phthalsäureanhydrid oder Adipinsäure.

3. Di-decylphthalat- oder Di-decyladipat-Gemisch gemäß Anspruch 2, dadurch gekennzeichnet, daß die Stufe a) in homogener Phase mit einem Katalysator aus Alkylaluminiumdihalogenid und einem Nickelsalz einer Fettsäure durchgeführt wird.